# EUROPÄISCHE PATENTANMELDUNG

(11) **EP 3 168 620 A1**
(43) Veröffentlichungstag der Anmeldung: **17.05.2017**
(21) Anmeldenummer: 15169620.0
(22) Anmeldetag: 28.05.2015
(51) Int. Cl.: G01N 35/00

(54) **ABFALLVERBRINGUNGSSYSTEM**

(71) Anmelder: Siemens Healthcare Diagnostics Products GmbH, 35041 Marburg (DE)
(72) Erfinder: Kalbfell, Heiko, 65232 Taunusstein (DE); Bernhard, Joachim, 61184 Karben (DE); Suder, Ingo, 66482 Zweibrücken (DE); Wilmes, Hugo, 65812 Bad Soden (DE)

(57) **Zusammenfassung**

Abfallverbringungssystem (1) zum Verbringen von Verbrauchsartikeln durch einen Abfallschacht (3) in einen Abfallbehälter (4) für Verbrauchsartikel umfassend einen Abfallschacht (3) mit einer Öffnung (6), wobei die Öffnung (6) eine Mehrzahl von Lamellen (8) zum teilweise oder vollständigen Verschließen der Öffnung (6) umfasst.

## Beschreibung

Gegenstand der Erfindung ist ein Abfallverbringungssystem für einen automatisch arbeitenden Analysator und liegt auf dem technischen Gebiet der automatischen Analysegeräte.

Zahlreiche Nachweis- und Analyseverfahren zur Bestimmung physiologischer Parameter in Körperflüssigkeitsproben oder anderen biologischen Proben werden heute automatisiert in großer Anzahl in automatischen Analysegeräten, auch so genannten in vitro-Diagnostiksystemen, durchgeführt.

Heutige Analysegeräte sind in der Lage, eine Vielzahl von Nachweisreaktionen und Analysen mit einer Probe durchzuführen. Um eine Vielzahl von Untersuchungen automatisiert durchführen zu können, sind diverse Vorrichtungen zum räumlichen Transfer von Messzellen, Reaktionsbehältern und Reagenzbehältern erforderlich, wie z.B. Transferarme mit Greiffunktion, Transportbänder oder drehbare Transporträder, sowie Vorrichtungen zum Transfer von Flüssigkeiten, wie z.B. Pipettiervorrichtungen. Die Geräte umfassen eine Steuereinheit, die mittels entsprechender Software dazu in der Lage ist, die Arbeitsschritte für die gewünschten Analysen weitgehend selbstständig zu planen und abzuarbeiten.

Viele der in derartigen automatisiert arbeitenden Analysegeräten verwendeten Analyseverfahren beruhen auf optischen Methoden. Diese Verfahren ermöglichen den qualitativen und quantitativen Nachweis von Analyten, d.h. der nachzuweisenden oder zu bestimmenden Substanzen in Proben. Die Bestimmung klinisch relevanter Parameter, wie zum Beispiel der Konzentration oder Aktivität eines Analyten, erfolgt vielfach, indem ein Teil einer Probe mit einem oder mehreren Testreagenzien in einem Reaktionsgefäß, welches auch die Messzelle sein kann, vermischt wird, wodurch z.B. eine biochemische Reaktion oder eine spezifische Bindungsreaktion in Gang gesetzt wird, die eine messbare Veränderung einer optischen oder anderen physikalischen Eigenschaft des Testansatzes bewirkt.

Es ist bekannt, dass in automatisch arbeitenden Analysatoren, die zur Untersuchung von biologischen Körperflüssigkeiten eingesetzt werden, die benötigten Reagenzien mittels einer Pipettiervorrichtung mit einer Pipettiernadel in eine Messküvette eingefüllt werden. Die Messküvette wird dabei mit einem Küvettengreifer innerhalb des automatischen Analysegerätes zu verschiedenen Positionen mittels eines Robotorarms automatisch verfahren, der Teil einer Robotorstation ist. Nach der Messung wird die benutzte Messküvette zur Entsorgung durch einen Abfallschacht in einen Abfallbehälter verbracht. Die benutzte Küvette enthält häufig noch Flüssigkeit. Die in der Küvette sich befindende Flüssigkeit soll nicht aus der Küvette heraus spritzen und den Analysator kontaminieren.

Die Messküvette wird zur Entsorgung durch den Abfallschacht in den Abfallbehälter mit dem Küvettengreifer zum Abfallschacht verfahren und in diesen verbracht. Dabei besteht die Gefahr, dass Flüssigkeit, die sich in den benutzten Küvetten befindet, aus den Küvetten heraus spritzt und den Analysator kontaminiert. Weiter ist die Kontaminationsgefahr für Personal, das das Analysegerät bedient, erheblich.

Aus der EP 2308588 A2 sind verschiedene Greifsysteme und Greifer für Küvetten bekannt, die z.B. einteilig und elastisch verformbar ausgestaltet sind und passiv funktionieren. Andere Greifer sind mehrteilig ausgestaltet und können aktiv Greifbewegungen ausführen.

Beispielsweise wird die Messküvette am Abfallschacht vom Küvettengreifer an einen weiteren Greifer übergeben, der als mehrteiliger elektrischer Greifer ausgestaltet ist und aktiv Greifbewegungen ausführen kann. Der leere Küvettengreifer kann weiter verfahren werden, um die nächste Messküvette zu prozessieren. Der elektrische Greifer wird mittels einer Steuerungssoftware über dem Abfallrohr geöffnet, und die benutzte Küvette fällt durch den Abfallschacht in den Abfallbehälter. Dies führt dazu, dass das Verbringen von benutzten Messküvetten vergleichsweise aufwändig, zeitintensiv und fehleranfällig ist. Besonders betrifft dies die Übergabe der Messküvette an den elektrischen Greifer und den elektrischen Greifer selbst. Dies führt zu einem höheren Kosten- und Zeitaufwand und erhöht die Fehleranfälligkeit in der Abarbeitung eine Analyse in einem automatischen Analysegerät erheblich.

Ähnliche Probleme wie bei den Küvetten ergeben sich auch bei anderen Verbrauchsartikeln wie z.B. austauschbaren Pipettenspitzen.

Es ist daher Aufgabe der Erfindung, ein Abfallverbringungssystem zur Verfügung zu stellen, welches die Kontaminationsgefahr für Personal, das das Analysegerät bedient, verringert. Weiter soll eine Reduktion des Kosten- und Zeitaufwands und eine niedrigere Fehleranfälligkeit in einem automatischen Analysegerät erreicht werden.

### Diese Aufgabe wird erfindungsgemäß durch die im Folgenden beschriebenen Gegenstände und Verfahren gelöst.

Es wurde gefunden, dass ein verbessertes Abfallverbringungssystem erreicht werden kann, wenn eine Mehrzahl von Lamellen zum teilweisen oder vollständigen Verschließen der Öffnung des Abfallschachts vorgesehen ist. Die Verbrauchsartikel können zwischen die Lamellen gesteckt werden und werden dann von diesen festgehalten.

Dies hat den Vorteil, dass Verspritzen von Flüssigkeit, die sich in den Verbrauchsartikeln befindet kann, vermieden wird und somit eine Senkung der Kontaminationsgefahr durch Verspritzen der Flüssigkeit erreicht wird. Weiter hat dies den Vorteil, dass die Lamellen zusätzliche mechanische und elektrische Komponenten, wie z.B. einen elektrischen Greifer ersetzen können und so elektrisch bewegte Teile eingespart werden können. Dies ermöglicht eine Reduktion des Kosten- und Zeitaufwands und eine niedrigere Fehleranfälligkeit in der Abarbeitung einer Analyse in einem automatischen Analysegerät.

Gegenstand der vorliegenden Erfindung ist ein Abfallverbringungssystem zum Verbringen von Verbrauchsartikeln durch einen Abfallschacht in einen Abfallbehälter, das Abfallverbringungssystem umfassend einen Abfallschacht mit einer Öffnung, wobei der Abfallschacht eine Mehrzahl von Lamellen zum teilweise oder vollständigen Verschließen der Öffnung umfasst.

In einer bevorzugten Ausführung ist die Mehrzahl von Lamellen zum teilweisen oder vollständigen Verschließen der Öffnung mit dem Abfallschacht verbunden.

Bevorzugt ist der Abfallschacht mindestens so lang wie die längste Ausdehnung der Verbrauchsartikel. Dies bewirkt, dass die Verbrauchsartikel nicht unmittelbar in den Abfallbehälter fallen, sondern zunächst sich vollständig im Abfallschacht befinden und vom Abfallschacht geleitet in den Abfallbehälter fallen. Weiter bewirkt dies, dass eine gewisse räumliche Trennung zwischen der Öffnung des Abfallschachts und dem Abfallbehälter erfolgt. Dies hat den Vorteil, dass ggf. noch in den Verbrauchsartikeln befindliche Flüssigkeit, die im Abfallbehälter aus den Verbrauchsartikeln austritt, nicht oder nur im verminderten Maß durch den Abfallaschacht hinaus gelangen kann durch z.B. Herausspritzen. Dies kann beispielsweise das Kontaminationsrisiko von Betriebs- und Servicepersonal reduzieren.

Bevorzugt ist der Querschnitt des Abfallschachts größer als die kleinste Ausdehnung, und kleiner als die größte Ausdehnung der Verbrauchsartikel. Somit ist beispielsweise bei Messküvetten, die eine zylindrische Form mit einer Höhe und einem Durchmesser aufweisen, wobei die Höhe größer ist als der Durchmesser, der Querschnitt des Abfallschachts bevorzugt größer als der Durchmesser und kleiner als die Höhe der Messküvetten. Dies hat den Vorteil, dass die Verbrauchsartikel den Abfallschacht nur in einer bestimmten Ausrichtung passieren können. Somit wird z.B. erreicht, dass ggf. noch in den Verbrauchsartikeln befindliche Flüssigkeit erst im Abfallbehälter aus den Verbrauchsartikeln austritt und nicht schon innerhalb des Abfallschachts. Dies kann beispielsweise das Kontaminationsrisiko von Betriebs- und Servicepersonal weiter reduzieren.

Bevorzugt handelt es sich bei den Verbrauchsartikeln um Küvetten oder um Pipettenspitzen.

Bevorzugt handelt es sich bei den Verbrauchsartikeln weiter um Produkte, die nur einmal verwendet und dann entsorgt bzw. ersetzt werden.

Insbesondere ist ein Abfallverbringungssystem zum Verbringen von Küvetten durch einen Abfallschacht in einen Abfallbehälter für Küvetten Gegenstand der vorliegenden Erfindung. Das Abfallverbringungssystem umfasst einen Abfallschacht mit einer Öffnung, der eine Mehrzahl von mit dem Abfallschacht verbundene Lamellen zum teilweise oder vollständigen Verschließen der Öffnung umfasst und wobei die Lamellen selbstrückstellend ausgestaltet sind.

Die erste benutzte Messküvette wird zur Öffnung zum Abfallschacht verfahren und dort mit dem Küvettengreifer zwischen die Lamellen gesteckt. Die Lamellen sind so ausgerichtet, dass sie eine Kraft auf die Messküvette ausüben und diese fest halten, so dass die benutzte Messküvette nicht in den Abfallschacht fällt. Der leere Küvettengreifer kann z.B. weiter verfahren werden um die nächste Messküvette zu prozessieren.

Die Lamellen sind dabei vorteilhafterweise so ausgerichtet und ausgestaltet, dass sie die Bewegung der Messküvette so einschränken, dass ein Herausspritzen von Flüssigkeit aus der Küvette verhindert wird. Weiter sind die Lamellen vorteilhafterweise aus einem elastischen Material und/oder elastisch mit dem Abfallschacht verbunden, um eventuell beim Kontakt der Messküvette mit den Lamellen auftretende Beschleunigungen der Messküvette zu dämpfen und so Herausspritzen von Flüssigkeit aus der Küvette auch dann zu verhindern, wenn es zu Beschleunigungen der Messküvette kommt.

Eine zweite benutzte Messküvette wird dann entsprechend auf die erste benutzte Messküvette gesetzt. Der Küvettengreifer drückt die erste benutzte Messküvette dabei mit der zweiten benutzten Messküvette senkrecht nach unten weiter in die Öffnung des Abfallschachts hinein. Mit weiteren benutzten Messküvetten wird entsprechend verfahren. Wird eine der benutzen Messküvette dabei hinreichend tief in die Öffnung des Abfallschachts hineingedrückt, wird diese Messküvette von den Lamellen freigegeben und fällt in den Abfallbehälter.

Vorzugsweise sind die Lamellen hinreichend elastisch um zu verhindern, dass die zurückfedernden Lamellen, die die unterste Messküvette freigegeben haben, nicht gegen die sich darüber befindende Küvette schlagen, da dies sonst zu einem Herausspritzen von Flüssigkeit aus dieser Küvette führen könnte.

Mittels der Ausgestaltung der Lamellen kann erreicht werden, dass die erste benutzte Messküvette bereits durch die zweite benutzte Messküvette hinreichend tief in die Öffnung des Abfallschachts hineingedrückt wird, um von den Lamellen freigegeben zu werden und in den Abfallbehälter zu fallen. Alternativ können die Lamellen so ausgestaltet sein, dass drei oder mehr benutzte Messküvetten auf die erste benutze Messküvette gesteckt werden müssen, bevor diese von den Lamellen freigegeben wird und in den Abfallbehälter fällt. Die entsprechende Ausgestaltung der Lamellen umfasst dabei beispielsweise deren Größe, Form und/oder Anordnung.

Vorteilhafterweise umfasst ein erfindungsgemäßes Abfallverbringungssystem einen Abfallschacht, einen Abfallbehälter und eine Halterung für den Abfallschacht, wobei Küvetten durch den Abfallschacht in den Abfallbehälter verbracht werden können und der Abfallschacht mittels der Halterung am Abfallbehälter befestigt werden kann.

Weiter umfasst das Abfallverbringungssystem vorteilhafterweise eine Robotorstation mit einem automatisch verfahrbaren Transferarm mit einem Küvettengreifer zum Verfahren von Küvetten innerhalb des Aktionsbereichs des automatisch verfahrbaren Transferarms.

Vorteilhafterweise sind der Abfallschacht und der Abfallbehälter so im Schwerefeld der Erde angeordnet, dass eine Küvette durch die erste Öffnung des Abfallschachts in den Abfallschacht verbracht werden kann und dann durch die Schwerkraft nach unten durch den Abfallschacht in den Abfallbehälter fällt.

Vorteilhafterweise umfasst das Abfallverbringungssystem mindestens ein Kunststoffspritzteil. Vorteilhafterweise sind der Abfallschacht, der Abfallbehälter, der Küvettengreifer und/oder der Lamelleneinsatz jeweils ein Kunststoffspritzteil.

Vorteilhafterweise wird der Abfallschacht besonders stabil ausgestaltet, und die Lamellen werden elastisch ausgestaltet. Dies hat den Vorteil, dass Beschleunigungskräfte auf die Messküvetten vermieden oder wenigstens minimiert werden.

In vorteilhafter Ausgestaltung bestehen die Lamellen aus Kunststoff und/oder Gummi. Dies ermöglicht eine besonders preiswerte Fertigung der Lamellen mit üblichen Herstellungsverfahren. In weiterer vorteilhafter Ausgestaltung sind die Lamellen aus Metall, z.B. Kupfer, Stahl, Aluminium.

In weiterer vorteilhafter Ausgestaltung sind die Lamellen mit dem Abfallschacht lösbar verbunden, z.B. durch Einklemmen der Lamellen in den Abfallschacht, mittels einer Klebeverbindung und/oder einer Schraubverbindung. Dies hat den Vorteil, dass die Lamellen, z.B. bei Verschleiß, einfach und kostengünstig ausgetauscht werden können, ohne dass der Abfallschacht oder weitere Teile des Abfallverbringungssystems mitausgetauscht werden müssen. Dies kann auch zu erheblichen Kostenersparnissen führen.

In bevorzugter Ausführungsform sind die Lamellen miteinander verbunden und bilden einen Lamelleneinsatz, der als ein Teil am Abfallschacht befestigt werden kann. Dies vereinfacht das Anbringen und Austauschen der Lamellen erheblich, da nur ein Lamelleneinsatz zu handhaben ist und nicht eine Vielzahl einzelner Lamellen.

In bevorzugter Ausführungsform sind die Lamellen im Querschnitt des Abfallschachts radial nach innen gerichtet. Dies hat den Vorteil, dass der Lamelleneinsatz besonders einfach und kostengünstig gefertigt werden kann. Weiter ist die Funktion des Lamelleneinsatzes unabhängig von der radialen Orientierung des Lamelleneinsatzes auf der Öffnung des Abfallschachts. Dies kann besonders bei Küvetten mit einem eckigen Querschnitt vorteilhaft sein, da solche Küvetten dann unabhängig von ihrer Orientierung relativ zum Abfallschacht bzw. des Lamelleneinsatzes in den Lamelleneinsatz gesteckt werden können und eine aufwändige Orientierung unterbleiben kann.

In weiterer bevorzugter Ausführungsform umfasst der Lamelleneinsatz mindestens einen Lamellenkranz. Dies hat den Vorteil, dass der Lamelleneinsatz einfach und kostengünstig hergestellt werden kann. Weiter kann der Lamelleneinsatz so besonders einfach einstückig gefertigt werden, was eine besonders einfache und kostengünstige Herstellung und Montage bzw. Demontage ermöglicht. Dies ist von besonderer Bedeutung, wenn der Lamelleneinsatz als Verschleißteil ausgebildet ist und regelmäßig ausgetauscht werden muss. Weiter werden Küvetten von einem Lamellenkranz besonders zuverlässig festgehalten, und es kann ein Kippen der Messküvette verhindert werden, was sonst zum Herauslaufen von Flüssigkeit aus der Messküvette führen könnte.

In besonders bevorzugter Ausführungsform umfasst der Lamelleneinsatz mindestens zwei übereinander angeordnete Lamellenkränze. Dies hat den Vorteil, dass Küvetten besonders zuverlässig festgehalten werden. Insbesondere werden die Küvetten von den Lamellenkränzen in einer senkrechten Position gehalten und stellen sich nicht schräg. Dies vermeidet besonders zuverlässig, dass Flüssigkeit aus einer benutzten Küvette unbeabsichtigt austreten kann.

In weiterer bevorzugter Ausführungsform sind die Lamellen einander benachbarter Lamellenkränze zueinander versetzt. Dies hat den Vorteil, dass Küvetten besonders leicht und wenig fehleranfällig in die Lamellenkränze gesteckt werden können. Weiter werden die Küvetten beim Einsetzen in die Lamellenkränze besonders akkurat bezüglich der Lamellenkränze ausgerichtet und zentriert. Dies ermöglicht ein besonders zuverlässiges Verbringen der Küvetten durch den Abfallschacht in den Abfallbehälter.

In weiterer bevorzugter Ausführungsform weist die Öffnung des Abfallschachts einen runden, ovalen, quadratischen, rechteckigen oder polygonen Querschnitt auf. Dies hat den Vorteil, dass die Form des Querschnitts der Öffnung an den Querschnitt der Küvetten angepasst werden kann. Dies ermöglicht ein besonders einfaches und zuverlässiges Verbringen der Küvetten durch den Abfallschacht in den Abfallbehälter. Weiter kann der Abfallschacht so besonders raumsparend ausgestaltet werden, was in automatischen Analysegeräten von besonderem Vorteil sein kann.

In einer anderen bevorzugten Ausführungsform umfasst der Abfallschacht im Bereich der Öffnung einen Öffnungsmechanismus für einen Küvettengreifer. Wenn eine Messküvette mittels eines Küvettengreifers zur Öffnung des Abfallschachts verfahren und dort mit dem Küvettengreifer in die Lamellen gesteckt wird, öffnet der Öffnungsmechanismus den Küvettengreifer gleichzeitig, so dass der Küvettengreifer die Küvette loslässt und ohne Küvette weiter verfahren werden kann, um weitere Küvetten zu prozessieren. Dies hat den Vorteil, dass Küvetten einerseits fest mit dem Küvettengreifer verbunden sind bei der Verfahrung und andererseits ohne größeren zusätzlichen Aufwand in der Öffnung des Abfallschachts vom Küvettengreifer freigegeben werden.

In weiterer bevorzugter Ausführungsform umfasst der Öffnungsmechanismus eine Struktur zum Öffnen eines Küvettengreifers, die in der Form ähnlich einem Prisma mit einem Dreieck als Grund- und Deckfläche ist. Dies hat den Vorteil, dass der Öffnungsmechanismus besonders einfach und kostengünstig implementiert werden kann. Weiter ist diese Ausgestaltung besonders vorteilhaft in Kombination mit einem einstückig gefertigten Küvettengreifer, der durch die dreieckige Struktur besonders leicht und effizient auseinandergedrückt werden kann, um die Küvette freizugeben.

Bevorzugt ist ein erfindungsgemäßes Abfallverbringungssystem für ein automatisches Analysegerät geeignet.

Ein anderer Gegenstand der Erfindung ist ein Abfallbehälter mit einem vorgenannten erfindungsgemäßen Abfallverbringungssystem. Dies hat den Vorteil, dass die benutzten Küvetten in dem Abfallbehälter gesammelt werden können und der weiteren Entsorgung bzw. Wiederverwertung im Rohstoffkreislauf zugeführt werden können.

Ein anderer Gegenstand der Erfindung ist ein Halter für einen vorgenannten erfindungsgemäßen Abfallschacht. Der Halter ist mit einem Abfallbehälter verbunden und erlaubt die Herstellung einer lösbaren Verbindung zwischen dem Abfallschacht und dem Abfallbehälter. Bevorzugt handelt es sich bei der lösbaren Verbindung um eine Steckverbindung, die ohne Werkzeugeinsatz per Hand hergestellt und gelöst werden kann. Dies hat den Vorteil, dass der Abfallschacht besonders leicht und schnell montiert und demontiert werden kann. Dies führt auch zu erheblichen Kostenvorteilen.

In besonders vorteilhafter Ausgestaltung wird die Steckverbindung zwischen Halter und Abfallschacht durch eine Drehbewegung des Abfallschachts arretiert. Dies hat den Vorteil, dass die lösbare Verbindung zwischen Halter und Abfallschacht besonders einfach und wenig fehleranfällig hergestellt und gelöst werden kann.

Bevorzugt ist das erfindungsgemäße Abfallverbringungssystem zur Verwendung in einem automatischen Analysegerät geeignet.

Ein anderer Gegenstand der Erfindung ist ein Analysegerät, welches ein vorgenanntes erfindungsgemäßes Abfallverbringungssystem umfasst. Das Analysegerät umfasst weiter vorteilhafterweise mindestens eine Pipettiereinheit und mindestens eine Messeinrichtung zum optischen und/oder elektronischen quantitativen Nachweis von mindestens einem Analyten in einer Probe.

Ein weiterer Gegenstand der Erfindung ist die Verwendung eines erfindungsgemäßen Abfallverbringungssystems zum Verbringen von Küvetten durch einen Abfallschacht in einen Abfallbehälter für Küvetten.

Ein weiterer Gegenstand der Erfindung ist die Verwendung eines erfindungsgemäßen Abfallverbringungssystems in einem automatischen Analysegerät zum Verbringen von Küvetten durch einen Abfallschacht in einen Abfallbehälter für Küvetten.

Ein weiterer Gegenstand der Erfindung ist ein Verfahren zum Verbringen von Küvetten in einen Abfallbehälter in einem erfindungsgemäßen automatischen Analysegerät umfassend einen Küvettengreifer, wobei der Küvettengreifer die Küvette zur Öffnung des Abfallschachts verfährt.

Unter einer "Probe" ist im Sinne der Erfindung das Material zu verstehen, dass die nachzuweisende Substanz (den Analyten) vermutlich enthält. Der Begriff "Probe" umfasst insbesondere biologische Flüssigkeiten von Menschen oder Tieren wie z.B. Blut, Plasma, Serum, Sputum, Exsudat, bronchoalveoläre Lavage, Lymphflüssigkeit, Synovialflüssigkeit, Samenflüssigkeit, Vaginalschleim, Feces, Urin, Liquor, aber auch z.B. durch Homogenisation oder Zelllyse für die photometrische, bevorzugt nephelometrische Bestimmung entsprechend aufgearbeitete Gewebe- oder Zellkulturproben. Ferner können auch z.B. pflanzliche Flüssigkeiten oder Gewebe, forensische Proben, Wasser- und Abwasserproben, Nahrungsmittel, Arzneimittel als Probe dienen, die ggf. vor der Bestimmung einer entsprechenden Probenvorbehandlung zu unterziehen sind.

Bei einem quantitativen Nachweis wird die Menge, die Konzentration oder die Aktivität des Analyten in der Probe gemessen. Von dem Begriff des "quantitativen Nachweises" sind auch semiquantitative Methoden umfasst, die nur die ungefähre Menge, Konzentration oder Aktivität des Analyten in der Probe erfassen oder nur zu einer relativen Mengen-, Konzentrations- oder Aktivitätsangabe dienen können. Unter einem qualitativen Nachweis ist der Nachweis des Vorhandenseins des Analyten in der Probe überhaupt oder das Anzeigen, dass die Menge, Konzentration oder Aktivität des Analyten in der Probe unterhalb oder oberhalb eines bestimmten oder mehrerer bestimmter Schwellenwerte liegt, zu verstehen.

Bei einer Messküvette handelt es sich beispielsweise um eine Küvette oder ein Reaktionsgefäß aus Glas, Kunststoff oder Metall. Vorteilhafterweise ist die Messküvette aus optisch transparenten Materialien gefertigt, was besonders beim Einsatz von optischen Analyseverfahren vorteilhaft sein kann.

Die Begriffe "Messküvette" und "Küvette" werden synonym verwendet.

### Die Erfindung wird anhand von Zeichnungen exemplarisch näher erläutert. Darin zeigen:

FIG 1 schematisch den Aufbau eines Abfallverbringungssystems (1) mit der Halterung (12), dem Küvettengreifer (11), dem Abfallschacht (3, 3') und dem Abfallbehälter (4),
FIG 2 schematisch den Abfallschacht (3) mit Öffnungsmechanismus (10) und ausgebautem Lamelleneinsatz (7),
FIG 3 schematisch eine Schnittzeichnung des Abfallschachts (3) mit im Küvettengreifer (11) gehaltener Küvette (2),
FIG 4 schematisch Details des Öffnungsmechanismus (10), des Küvettengreifers (11) und des Abfallschachts (3) von schräg oben,
FIG 5 schematisch eine durch die Lamellenkränze (9, 9') festgehaltene erste Messküvette (2) und eine zweite Messküvette (2') im Küvettengreifer (11),
FIG 6 schematisch die mit dem Abfallbehälter (4) verbindbare Halterung (12) mit gelöstem Abfallschacht (3).

### Gleiche Teile sind in allen Figuren mit denselben Bezugszeichen versehen.

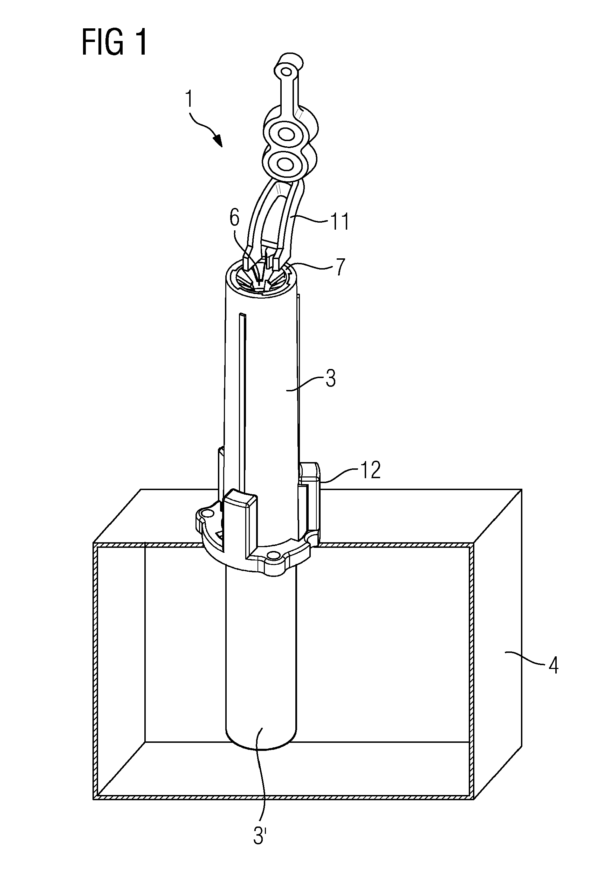

Das Abfallverbringungssystem (1) gemäß FIG 1 ist eingebettet in ein nicht näher dargestelltes Analysegerät, welches zur Ausführung einer Vielzahl von Analysen von Proben ausgestaltet ist. Dazu umfasst das automatische Analysegerät eine Vielzahl von nicht gezeigten Pipettiereinrichtungen und Transporteinrichtungen sowie weiterhin eine Steuereinheit zum automatisierten Auswerten der Analysen.

Das Abfallverbringungssystem (1) umfasst den Abfallschacht (3) mit der Öffnung (6), in der der Lamelleneinsatz (7) lösbar eingesetzt ist. Weiter umfasst das Abfallverbringungssystem (1) die Halterung (12), einen Abschnitt des Abfallschachts (3') und den Abfallbehälter (4) sowie den Küvettengreifer (11). Der Abschnitt des Abfallschachts (3') befindet sich innerhalb des Abfallbehälters (4). Die Halterung (12) ist am Abfallbehälter (4) befestigt und ist mit dem Abfallschacht (3) lösbar verbunden. Der Küvettengreifer (11) ist einstückig ausgestaltet und als Plastikspritzteil gefertigt.

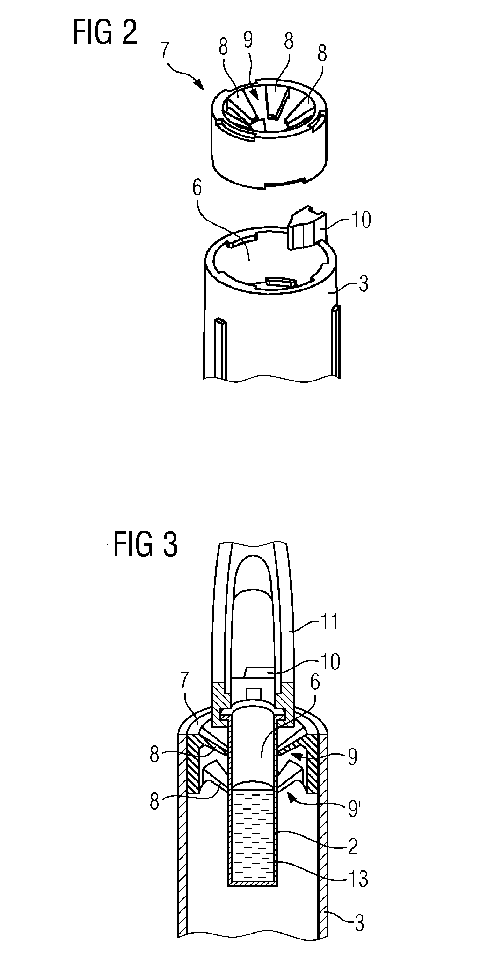

FIG 2 zeigt den Abfallschacht (3) mit der Öffnung (6) und dem Öffnungsmechanismus (10), der in der Form ähnlich einem regulären Prisma mit einem Dreieck als Grund- und Deckfläche ausgestaltet ist. Weiter zeigt FIG 2 den Lamelleneinsatz (7) mit Lamellen (8), die einen Lamellenkranz (9) bilden. Der Lamelleneinsatz (7) ist mit dem Abfallschacht (3) lösbar verbindbar, indem der Lamelleneinsatz (7) in den oberen Teil des Abfallschachts (3) wenigstens teilweise eingeführt wird.

FIG 3 zeigt den oberen Bereich des Abfallschachts (3) mit dem eingesetzten Lamelleneinsatz (7). Der Lamelleneinsatz (7) umfasst zwei übereinander angeordnete Lamellenkränze (9, 9'), die jeweils von einer Vielzahl von Lamellen (8) gebildet werden. In den Lamelleneinsatz (7) ist eine Küvette (2) mittels des Küvettengreifers (11) teilweise eingeführt. In der Küvette (2) befindet sich Flüssigkeit (13). Weiter ist der Öffnungsmechanismus (10) dargestellt. Der Küvettengreifer (11) weist zwei Arme auf, die von dem Öffnungsmechanismus (10) gespreizt werden können, der dann die gegriffene Küvette freigibt. Die Spreizung erfolgt durch Bewegung des Küvettengreifers in Richtung in die Bildebene hinein (nicht dargestellt).

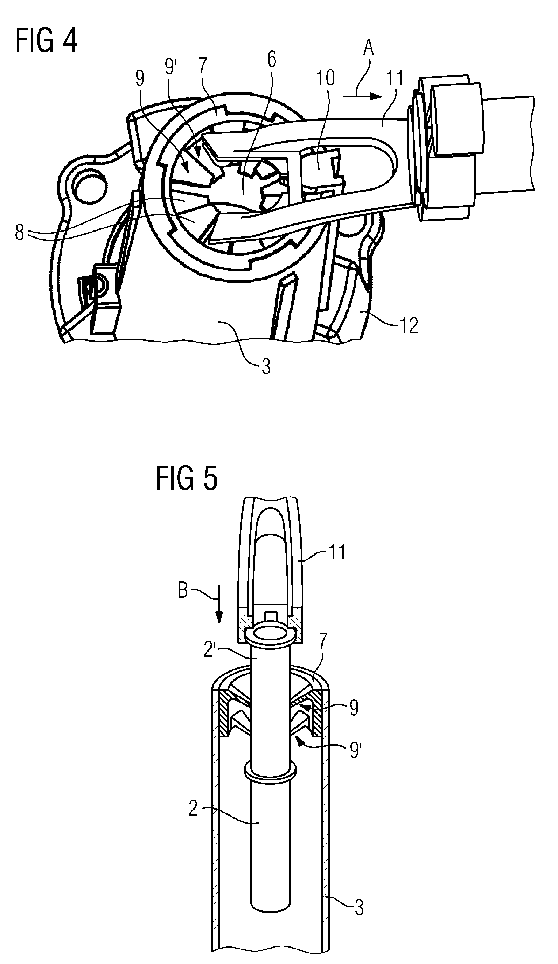

FIG 4 zeigt den oberen Bereich des Abfallschachts (3) mit dem eingesetzten Lamelleneinsatz (7) und dem Öffnungsmechanismus (10). Der Lamelleneinsatz (7) umfasst zwei übereinander angeordnete Lamellenkränze (9, 9'), die jeweils von einer Vielzahl von Lamellen (8) gebildet werden. Der Küvettengreifer (11) befindet sich über der Öffnung (6). Durch Bewegung des Küvettengreifers (11) in Richtung A werden die beiden Arme des Küvettengreifers (11) gespreizt. Hierdurch würde eine vom Küvettengreifer (11) festgehaltene Küvette (2) (nicht gezeigt) freigegeben werden. Weiter zeigt FIG 4 die Halterung (12).

FIG 5 zeigt eine Schnittzeichnung des oberen Bereichs des Abfallschachts (3) mit dem eingesetzten Lamelleneinsatz (7) und dem Öffnungsmechanismus (10). Der Lamelleneinsatz (7) umfasst zwei übereinander angeordnete Lamellenkränze (9, 9'), die jeweils von einer Vielzahl von Lamellen (8) gebildet werden. Eine erste Küvette (2) befindet sich im Lamelleneinsatz (7) und wird von den Lamellen (8) der Lamellenkränze (9, 9') festgehalten. Der Küvettengreifer (11) hält eine zweite Küvette (2') fest und drückt die erste Küvette (2) mittels der zweiten Küvette (2') in Richtung B durch die Lamellenkränze (9, 9') hindurch nach unten, so dass die erste Küvette (2) durch den Abfallschacht (3) nach unten in den Abfallbehälter (4) (nicht gezeigt) fallen kann.

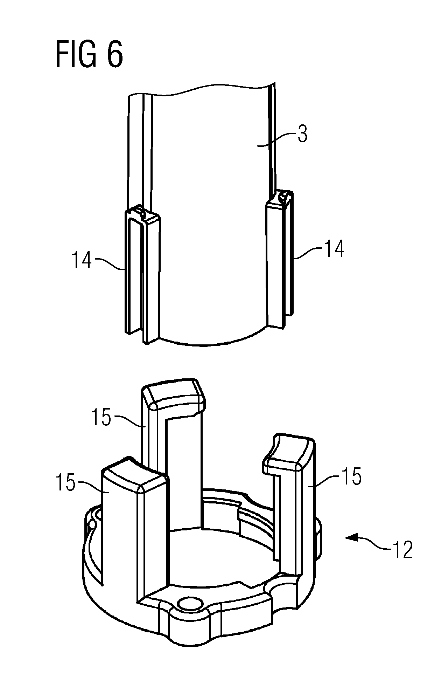

FIG 6 zeigt den unteren Teil des Abfallschachts (3) mit den ersten Halteelementen (14), die jeweils als hervorstehender Steg seitlich am Abfallschacht (3) an dessen unteren Ende außen hervorstehen. Insgesamt weist der Abfallschacht (3) drei erste Haltelemente (14) auf, die im Winkelabstand von etwa 120 Grad um den Abfallschacht (3) herum angeordnet sind. Die Halterung (12) weist zu den ersten Halteelementen (14) komplementäre zweite Halteelemente (15) auf. Der Abfallschacht (3) lässt sich mittels der ersten (14) und zweiten (15) Halteelemente mit der Halterung lösbar verbinden. Durch Verdrehung lässt sich die lösbare Verbindung arretieren.

### Bezugszeichenliste

- 1: Abfallverbringungssystem
- 2, 2': Küvette
- 3, 3': Abfallschacht
- 4: Abfallbehälter
- 6: Öffnung
- 7: Lamelleneinsatz
- 8: Lamellen
- 9, 9': Lamellenkranz
- 10: Öffnungsmechanismus
- 11: Küvettengreifer
- 12: Halterung
- 13: Flüssigkeit
- 14: Erstes Halteelement
- 15: Zweites Halteelement
- A, B: Richtung

## Patentansprüche

1. Abfallverbringungssystem (1) zum Verbringen von Verbrauchsartikeln durch einen Abfallschacht (3) in einen Abfallbehälter (4), das Abfallverbringungssystem (1) umfassend einen Abfallschacht (3) mit einer Öffnung (6), **dadurch gekennzeichnet, dass**
der Abfallschacht (3) eine Mehrzahl von selbstrückstellenden Lamellen (8) zum teilweisen oder vollständigen Verschließen der Öffnung (6) umfasst.

2. Abfallverbringungssystem (1) nach Anspruch 1, wobei die Verbrauchsartikel Küvetten (2) oder Pipettenspitzen sind.

3. Abfallverbringungssystem (1) nach einem der vorhergehenden Ansprüche, wobei die Lamellen (8) aus einem elastischen Material bestehen und/oder elastisch mit dem Abfallschacht verbunden sind.

4. Abfallverbringungssystem (1) nach einem der vorhergehenden Ansprüche, wobei die Lamellen (8) aus Kunststoff, Gummi und/oder Metall bestehen.

5. Abfallverbringungssystem (1) nach einem der vorhergehenden Ansprüche, wobei die Lamellen (8) mit dem Abfallschacht (3) lösbar verbunden sind.

6. Abfallverbringungssystem (1) nach einem der vorangehenden Ansprüche, wobei die Lamellen (8) einen Lamelleneinsatz (7) bilden.

7. Abfallverbringungssystem (1) nach einem der vorangehenden Ansprüche, wobei die Lamellen (8) im Querschnitt des Abfallschachts (3) nach innen gerichtet sind.

8. Abfallverbringungssystem (1) nach einem der vorangehenden Ansprüche, wobei die Lamellen (8) mindestens einen Lamellenkranz (9) bilden.

9. Abfallverbringungssystem (1) nach einem der vorausgehenden Ansprüche, wobei die Lamellen (8) mindestens zwei übereinander angeordnete Lamellenkränze (9, 9') bilden.

10. Abfallverbringungssystem (1) nach Anspruch 9, wobei die Lamellen (8) einander benachbarter Lamellenkränze (9, 9') zueinander versetzt sind.

11. Abfallverbringungssystem (1) nach einem der vorhergehenden Ansprüche, wobei am Abfallschacht (3) ein Öffnungsmechanismus (10) für einen Küvettengreifer (11) angebracht ist.

12. Abfallverbringungssystem (1) nach Anspruch 11, wobei der Öffnungsmechanismus (10) eine Struktur umfasst, die in der Form ähnlich eines Prismas mit einem Dreieck als Grund- und Deckfläche ist.

13. Automatisches Analysegerät umfassend ein Abfallverbringungssystem (1) nach einem der Ansprüche 1 bis 12.

14. Automatisches Analysegerät nach Anspruch 13 umfassend einen Küvettengreifer (11).

15. Verfahren zum Verbringen von Küvetten (2) in einen Abfallbehälter (4) in einem automatischen Analysegerät nach Anspruch 14, wobei der Küvettengreifer (11) die Küvette zur Öffnung (6) des Abfallschachts (3) verfährt.
